**Europäisches Patentamt**

**European Patent Office**

**Office européen des brevets**

(19)

(11) Numéro de publication : **0 205 371 B1**

# FASCICULE DE BREVET EUROPEEN

(12)

(45) Date de publication du fascicule du brevet :
**06.02.91 Bulletin 91/06**

(21) Numéro de dépôt : **86401063.2**

(22) Date de dépôt : **20.05.86**

(51) Int. Cl.⁵ : **C12N 15/56,** C12N 9/28, C12N 1/20, // (C12N1/20, C12R1:125)

(54) **Vecteurs d'expression de l'alpha-amylase dans bacillus subtilis, souches obtenues et procédé de préparation d'alpha-amylase.**

(30) Priorité : 22.05.85 FR 8507676

(43) Date de publication de la demande :
17.12.86 Bulletin 86/51

(45) Mention de la délivrance du brevet :
06.02.91 Bulletin 91/06

(84) Etats contractants désignés :
**AT BE CH DE GB IT LI LU NL SE**

(56) Documents cités :
EP-A- 0 117 823
FR-A- 2 537 602
FEMS MICROBIOLOGY LETTERS, vol. 21, 1984, pages 353-358, Federation of European Microbiological Societies; P. JOYET et al.: "Cloning of a thermostable alpha-amylase gene from Bacillus licheniformis and its expression in Escherichia coli and Bacillus subtilis"
IDEM
GENE, vol. 19, 1982, pages 277-284, Elsevier Biomedical Press, NL; B. NIAUDET et al.: "Insertional mutagenesis in Bacillus subtilis: mechanism and use in gene cloning"
JOURNAL OF BACTERIOLOGY, vol. 153, no. 3, mars 1983, pages 1424-1431, American Society for Microbiology, US; P. GAY et al.: "Cloning structural gene sacB, which codes for exoenzyme levansucrase of Bacillus subtilis: Expression of the gene in Escherichia coli"

(56) Documents cités :
MOL. GEN. GENET., vol. 191, 1983, pages 138-144, Springer Verlag; M .STEINMETZ et al.: "Analyse génétique de sacB, gène de structure d'une enzyme secrétée, la lévane-saccharase de Bacillus subtilis Marburg"

(73) Titulaire : **CENTRE NATIONAL DE LA RECHERCHE SCIENTIFIQUE (CNRS)**
**15, Quai Anatole France**
**F-75007 Paris (FR)**

(72) Inventeur : **Levin, Daniel**
**9, rue des Fêtes**
**F-75019 Paris (FR)**
Inventeur : **Joyet, Philippe**
**47, rue Fondary**
**F-75015 Paris (FR)**
Inventeur : **de Louvencourt, Laurence**
**105, rue de Grenelle**
**F-75006 Paris (FR)**
Inventeur : **Aymerich, Stéphane**
**6, rue Levert**
**F-75020 Paris (FR)**
Inventeur : **Le Reverend, Brigitte**
**118, avenue Jean Jaurès**
**F-92120 Montrouge (FR)**
Inventeur : **Steinmetz, Michel**
**100 Bis, rue des Pyrénées**
**F-75020 Paris (FR)**
Inventeur : **Heslot, Henri**
**29, rue Rousselet**
**F-75007 Paris (FR)**

(74) Mandataire : **Warcoin, Jacques et al**
**Cabinet Régimbeau 26, avenue Kléber**
**F-75116 Paris (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Sous le terme générique d'amylase, on désigne toute activité enzymatique capable d'hydrolyser tout ou partie des liaisons $\alpha$1-4 ou $\alpha$1-6 de l'amidon.

Dans la pratique industrielle, on utilise une $\alpha$-amylase, dite enzyme liquéfiante, afin d'hydrolyser l'amidon après l'avoir transformé en empois par chauffage à une température supérieure à 95°C. L'usage d'une $\alpha$-amylase thermostable présente un intérêt évident dans la mesure où elle évite d'avoir à refroidir l'empois avant de l'y ajouter.

L'$\alpha$-amylase thermostable commercialisée sous le nom de Termamyl par la firme danoise NOVO est produite par fermentation d'une souche de Bacillus licheniformis. Cette enzyme est sécrétée dans le milieu de culture. En l'absence de tout protecteur (ion $Ca^{++}$ et amidon) l'enzyme conserve 90% de son activité après 10 minutes à 80°C. En présence d'ions $Ca^{++}$ 0,1 M, l'enzyme est stable à 100% à 90°C pendant 15 minutes (1).

Dans les brevets d'invention n° 82 16099 (déposé le 24 septembre 1982) et n° 82 20904 (déposé le 13 décembre 1982) on décrit le clonage du gène de B. licheniformis codant pour l'$\alpha$-amylase thermostable et son expression chez Escherichia coli et chez Bacillus subtilis, le gène étant localisé sur un plasmide multicopies. Synthétisée par ces deux microorganismes, l'$\alpha$-amylase conserve toute ses propriétés inchangées : poids moléculaire, thermostabilité, activité spécifique en fonction du pH, propriétés immunologiques.

Chez B. licheniformis, la sécrétion de l'$\alpha$-amylase se produit pour l'essentiel en phase exponentielle tardive et en phase stationnaire. De plus, la synthèse est réprimée par la présence, dans le milieu de culture, de glucose et d'autres glucides qui exercent un effet de répression catabolique (2, 3).

La présente invention repose sur la possibilité de s'affranchir de ces limitations en mettant le gène de l'$\alpha$-amylase sous le contrôle du promoteur de la levane saccharase (LS dans ce qui suit). Cette enzyme (voir brevet n° 83 03320 déposé le 1er mars 1983) est sécrétée dans le milieu de culture par B. subtilis, après induction par le saccharose, pendant la phase exponentielle de croissance. La LS codée par le gène sacB, est soumise au contrôle d'un locus contigu sacR (promoteur/opérateur). L'expression de sacB est aussi contrôlée entre autres par les produits des gènes sacS et sacU. Certains allèles de sacU (allèles sacU^h) peuvent conduire à des niveaux d'expression très élevés du gène sacB. Chez ces mutants sacU^h la LS représente, après induction, jusqu'à 7% du total des protéines cellulaires et jusqu'à 95% des protéines extracellulaires (4, 5). On notera qu'il existe des mutants constitutifs (sacR^c, sacS^c) qui produisent la LS constitutivement, c'est-à-dire en l'absence du saccharose inducteur.

Comme on le verra dans les exemples ci-dessous, il peut être intéressant d'utiliser, pour exprimer nos constructions, des souches de B. subtilis possédant des allèles mutés des loci sacR, sacS et sacU.

L'un des objectifs de la présente invention est la mise au point de vecteurs assurant la production d'$\alpha$-amylase thermostable dans des conditions de culture et de milieu où elle n'est pas normalement produite par le microorganisme d'origine. La possibilité de s'affranchir de la répression catabolique par le glucose, de bénéficier d'une induction par le saccharose et de sécréter l'enzyme en phase exponentielle plutôt qu'en phase stationnaire paraît présenter des avantages. On peut en effet envisager de cultiver nos souches sur des sous-produits tels que les mélasses et réaliser des fermentations en continu, réputées économiquement et techniquement avantageuses.

La présente invention concerne des vecteurs de clonage et d'expression de l'$\alpha$-amylase dans Bacillus présentant la structure :

– sacR – amy

mettant le gène de l'$\alpha$-amylase (amy) sous le contrôle du promoteur/opérateur sacR de la lévane saccharase de B. subtilis, ce locus pouvant être sous la forme sauvage sacR^+ ou sous une forme mutée sacR^c (synthèse constitutive).

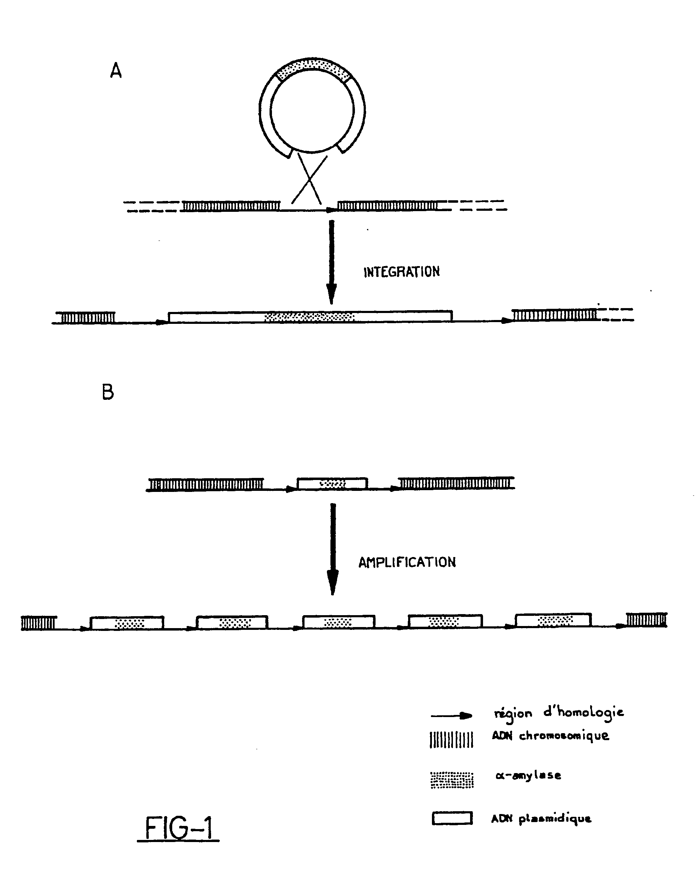

Les vecteurs qui assurent l'expression de l'$\alpha$-amylase chez B. subtilis peuvent être auto-réplicatifs multicopies ou bien encore être incapables de se répliquer chez l'organisme hôte. Dans ce derniers cas, le maintien de l'information génétique est assuré par intégration du vecteur dans le chromosome de la souche hôte de B. subtilis. On notera que de tels vecteurs intégrés sont nettement plus stables que leurs homologues contenant une origine de réplication (L. Jannière) (7). L'intégration dépend de la présence dans le vecteur de séquences d'ADN homologues de séquences chromosomiques. Ces dernières peuvent être elles-mêmes des séquences plasmidiques que l'on a intégrées au préalable dans le chromosome. L'intégration du plasmide non réplicatif peut s'effectuer par un "crossing-over" unique entre les régions homologues (6). On obtient alors (figure 1A) une duplication du fragment homologue qui encadre le gène de l'$\alpha$-amylase placé sous le contrôle du promoteur/opérateur sacR de la LS. Cette structure peut être amplifiée et conduit à des répétitions en tandem (7), voir figure 1B ; dans certaines limites, variables selon le contexte génétique, l'expression de la région amplifiée peut en être augmentée.

Avec certains vecteurs peut se produire un double "crossing-over" conduisant alors à une substitution de la région concernée (exemple 4) en particulier lorsque la structure est la suivante :

$$- Ha - sacR - \underline{amy} - Hb -$$

Ha et Hb étant des séquences homologues du chromosome.

Les vecteurs décrits dans la présente invention mettent le gène de l'α-amylase thermostable de B. licheniformis sous le contrôle de la région promoteur/opérateur sacR de la lévane saccharase de B. subtilis. Les vecteurs comportent au minimum :

1) L'essentiel ou la totalité du gène de structure de l'α-amylase.

2) Le locus sacR nécessaire à l'expression de sacB.

3) Eventuellement, une partie du gène de structure sacB de la lévane saccharase.

4) Un segment d'ADN d'un plasmide doté du réplicateur de ColEI capable de se répliquer dans E. coli, mais pas chez B. subtilis. Cet ADN porte un ou plusieurs gènes de résistance à des antibiotiques dont au moins un s'exprime chez chacun de ces hôtes.

5) Un ou plusieurs segments d'ADN homologue(s) de séquences présentes dans le chromosome de B. subtilis et permettant l'intégration du plasmide.

La structure des vecteurs peut être variée, il peut s'agir d'une structure :

$$- sacR - sacB' - \underline{amy} -$$

sacB' étant tout ou partie du gène de la LS,

le gène amy étant fusionné en phase avec sacB ;

ou bien d'une structure :

$$- sacR - sacB' - c.st. - SD - \underline{amy} -$$

sacB' représente une partie du gène sacB,

c.st. est un codon stop,

SD est la séquence de Shine-Dalgarno.

Un certain nombre des plasmides de ce type, décrits dans les exemples qui suivent, ont été construits dans E. coli. De plus, ont été construits des dérivés de ces derniers, capables de réplication autonome chez B. subtilis et comportant, outre les segments (1), (2), (3), (4), une origine de réplication fonctionnelle chez B. subtilis.

La présente invention concerne également les souches de Bacillus transformées par les vecteurs décrits précédemment, qu'ils soient auto-réplicatifs ou intégratifs.

La présente invention concerne également un procédé de préparation d'α-amylase dans lequel on cultive sur un milieu de croissance une souche de Bacillus transformée, éventuellement en présence d'un agent inducteur tel que le saccharose.

L'invention concerne, enfin, l'α-amylase obtenue par la mise en oeuvre du procédé précédent.

## EXEMPLE 1

### Construction d'un vecteur de clonage codant pour une protéine hybride LS-α-amylase thermorésistante et régulée comme la LS

A partir des séquences connues (8, 9) de la lévane saccharase chez B. subtilis et de l'α-amylase de B. licheniformis, on peut localiser les sites de coupures par diverses enzymes de restriction et diriger la fusion des deux gènes de telle sorte que le cadre de lecture du premier sacB soit en phase avec celui de l'α-amylase.

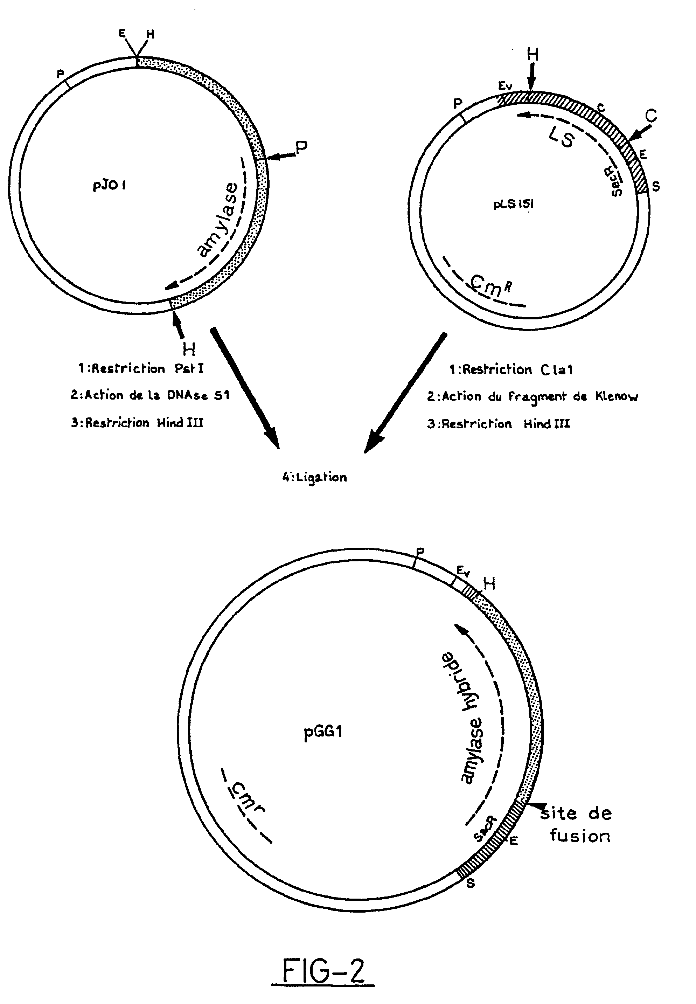

La fusion qui a été réalisée est décrite dans la figure 2.

Le plasmide pJ01 (10) est coupé par l'enzyme PstI puis soumis à l'action de la nucléase S1. On obtient ainsi un bout franc.

Le plasmide pLS 151 (8) est coupé par l'enzyme ClaI, puis traité par le fragment Klenow de l'ADN polymérase I. On obtient ainsi des bouts francs.

Dans une deuxième phase, les deux plasmides sont coupés par HindIII, mélangés et soumis à ligation par la ligase de T4.

L'un des plasmides hybrides obtenus contient le segment PstI-HindIII du gène de l'α-amylase, privé de son promoteur et inséré en aval du promoteur et du début du gène de la lévane saccharase.

## EXEMPLE 2

### Variante de l'exemple 1 permettant d'obtenir une plus grande variété de fusions LS-amy

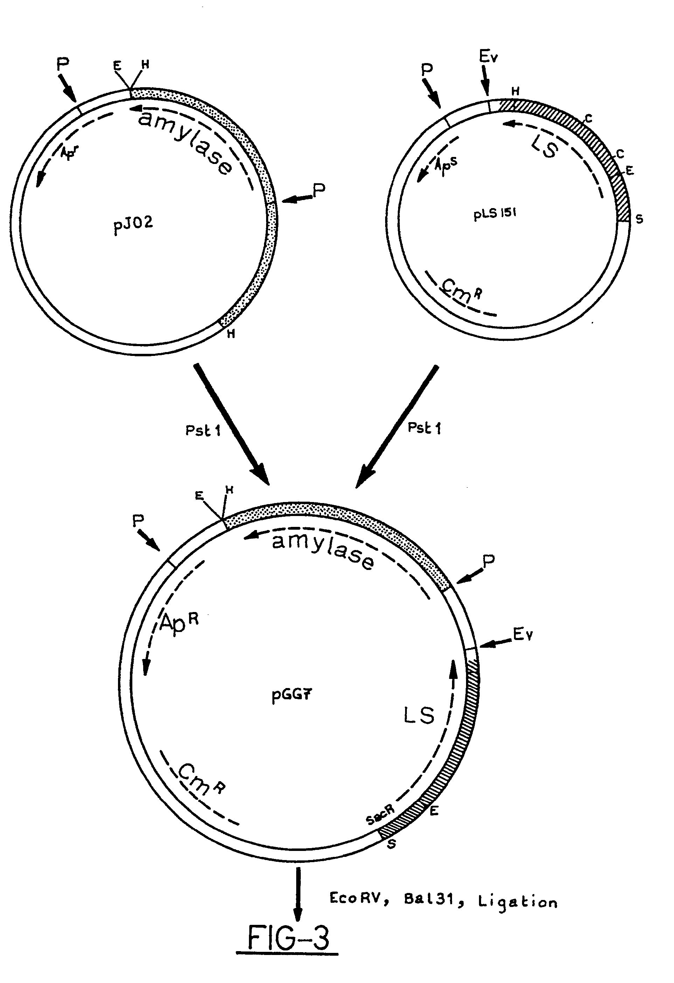

Un plasmide portant en tandem un gène sacB entier et un gène amy dépourvu de promoteur et de séquence signal a été construit (figure 3). Ce plasmide exprime la LS (phénotype sacᴿ), mais pas l'amylase (amy⁻).

Ce plasmide est soumis à l'action de l'exonucléase Bal31 après avoir été linéarisé en son site EcoRV. Si l'action de Bal31 est suffisamment prolongée, elle peut fusionner les gènes sacB et amy. La population de clones résultants peut être enrichie en produits de fusion par la sélection des clones de E. coli insensibles au saccharose chez lesquels le gène sacB est tronqué (phénotype sacᴿ).

Pour peu que ces fusions soient en phase, un gène hybride sera produit. Si la protéine résultante est stable et que l'action de Bal31 n'ait pas délété des régions essentielles à l'activité amylolytique, une amylase hybride (contenant une partie N-terminale LS) est produite sous le contrôle de la région sacR.

Les plasmides décrits dans l'exemple 1 sont donc des cas particuliers de ceux de l'exemple 2.

## EXEMPLE 3

### Construction d'un vecteur codant pour une amylase identique à celle de B. licheniformis, mais régulée par sacR

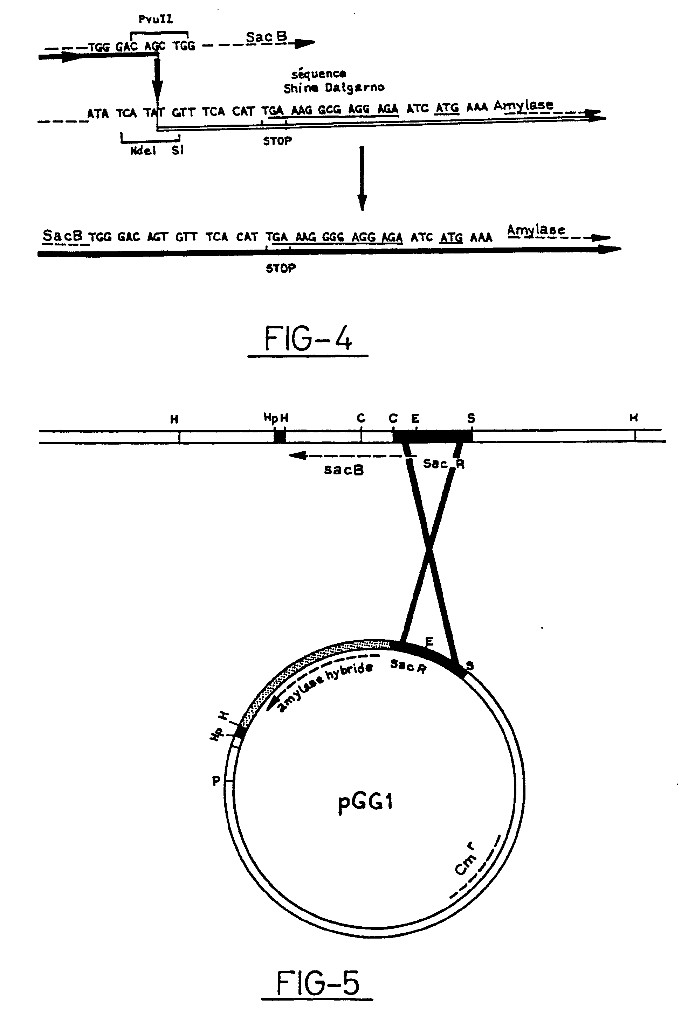

Le plasmide pJ01 est coupé par NdeI, traité par la nucléase S1, puis par HindIII. On notera (figure 4) qu'un site NdeI est situé après le promoteur de l'α-amylase, mais avant la séquence Shine-Dalgarno de fixation des ribosomes. Le gène de structure de l'α-amylase (y compris sa séquence signal) reste donc intact.

Le plasmide pLS151 est coupé par PvuII, puis par HindIII.

Les deux plasmides ainsi traités sont alors mélangés et ligués par la ligase T4. Un des plasmides hybrides résultant de ces opérations contient la totalité du gène de structure de l'α-amylase, séquence signal comprise, précédée d'une séquence Shine-Dalgarno, d'un codon stop et enfin de la région sacR suivie d'une fraction du gène de structure de la LS.

Introduit dans B. subtilis, un tel plasmide produira un ARN messager sous le contrôle de sacR, transcrit d'une fraction du gène sacB et de la totalité du gène l'α-amylase. En raison de la présence du codon d'arrêt de traduction d'une part, et de la séquence Shine-Delgarno d'autre part, on s'attend à ce que l'α-amylase produite soit identique à celle de B. licheniformis, mais soumise à la même régulation que la LS.

## EXEMPLE 4

### Construction d'un vecteur codant pour une α-amylase capable de s'intégrer à la place de sacB dans le chromosome de B. subtilis

Les constructions plasmidiques décrites dans les exemples 1 et 3 sont telles que le gène de l'α-amylase (hybride ou non) est précédé en amont par la région sacR et éventuellement du début du gène sacB. Ces séquences ont une longueur suffisante pour que le plasmide puisse s'intégrer à haute fréquence et par "crossing-over" simple au niveau des séquences chromosomiques homologues. Par contre, en aval du gène de l'α-amylase, la séquence d'homologie est courte, de l'ordre de 200 nucléotides : un "crossing over" à ce niveau sera donc rare. C'est pourquoi les évènements d'intégration résulteront pour l'essentiel de "crossing-over" simples dans la région amont, ce qui laisse intact un gène chromosomique sacB, en tandem avec le gène amy introduit (figure 5).

Si l'on veut substituer au gène sacB celui de l'α-amylase (hybride ou non), il faut introduire en aval du gène amy, dans les plasmides décrits dans les exemples 1 et 3, une séquence suffisamment longue, homologue à la région située normalement en aval du sacB.

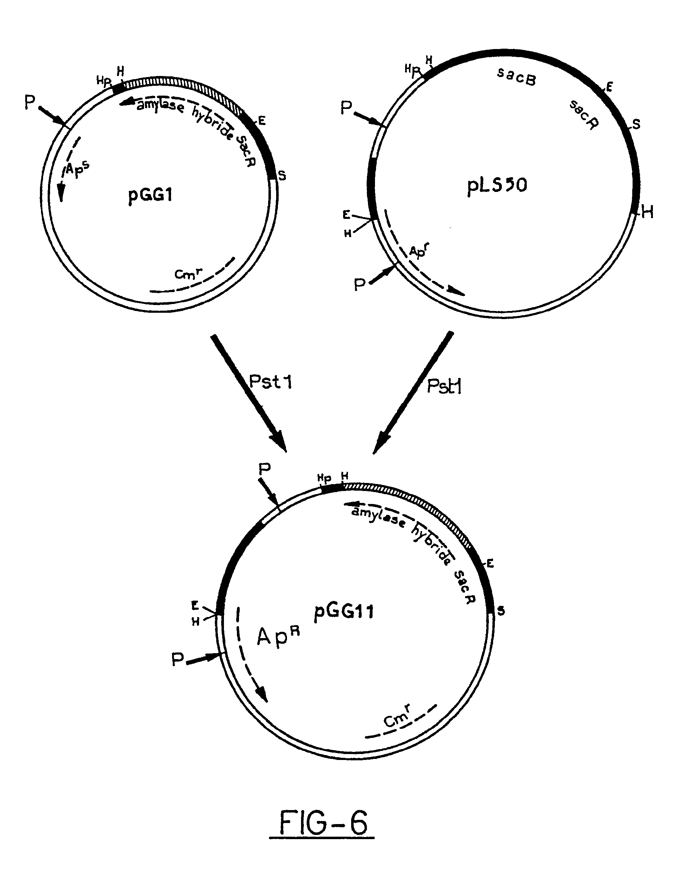

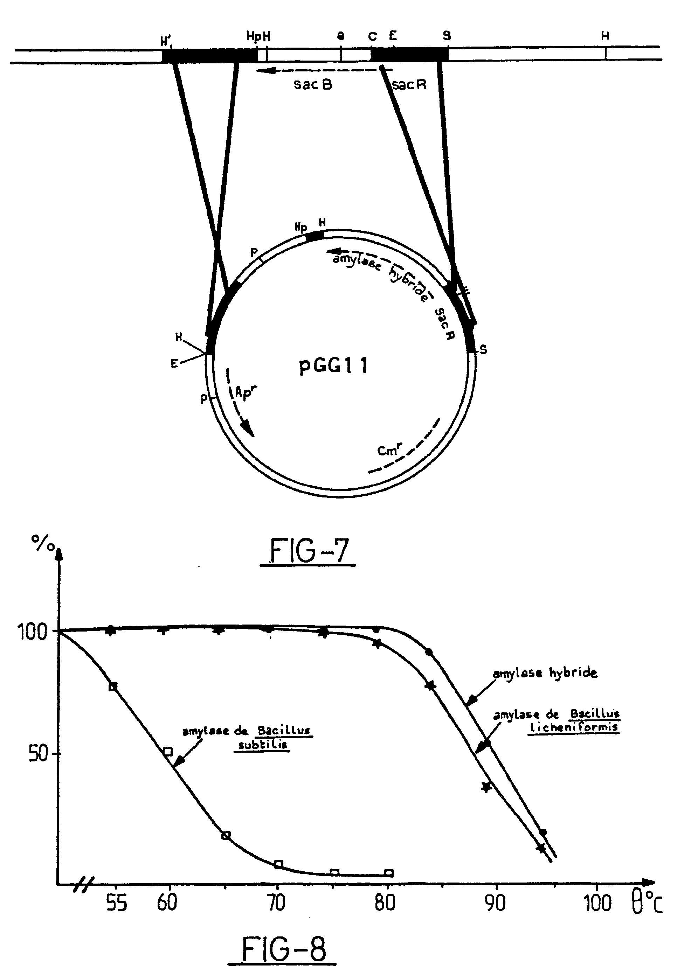

A cet effet, les plasmides des exemples 1 ou 3 sont linéarisés par l'enzyme de restriction PstI. On insère ensuite dans ce site un segment de restriction PstI-PstI issu du plasmide pLS50 (figure 6). Le plasmide résultant contient alors, de part et d'autre du gène de l'α-amylase, deux régions d'homologie d'une longueur suffisante pour permettre une intégration par double "crossing-over". Parmi les clones de B. subtilis transformés par ces plasmides, certains sont délétés pour tout ou partie du gène sacB lequel est remplacé par le gène hybride (figure 7).

Des dérivés des plasmides de l'exemple 2 présentant des propriétés analogues peuvent être construits en insérant, au site HindIII situé en aval du gène amy, le petit fragment HindIII de pLS50.

## EXEMPLE 5

Propriétés des α-amylases produites par les vecteurs décrits dans les exemples 1, 2 et 3

On a utilisé la technique des "minicells" pour déterminer le poids moléculaire des amylases produites chez E. coli.

Cette technique a montré qu'il s'agit bien, dans l'exemple 1, de protéines hybrides dont la taille est accrue et, dans l'exemple 3, d'une α-amylase identique à celle de B. licheniformis.

Construction des souches de B. subtilis ayant intégré des plasmides hybrides codant pour l'α-amylase

Les plasmides décrits dans les exemples 1, 2 et 3 possèdent un gène de résistance au chloramphénicol (CAT) présent dans pLS 151 (8), issu de pC194 (11) qui s'exprime chez B. subtilis et E. coli.

Pour ceux de ces plasmides qui sont non-réplicatifs chez B. subtilis, on a sélectionné leur intégration dans le chromosome de B. subtilis.

Les souches réceptrices peuvent bien entendu posséder divers allèles du locus sacR ou des gènes sacS et sacU. Les plasmides que l'on a décrits et qui expriment l'α-amylase sous le contrôle de sacR, peuvent être introduits dans les souches réceptrices, ce qui permet un grand nombre de combinaisons différentes. On ne décrit que quelques cas dans les exemples qui suivent.

## EXEMPLE 6

Construction d'une souche de B. subtilis exprimant l'α-amylase par intégration d'un des plasmides des exemples 1, 2 ou 3 à la suite d'un "crossing-over" simple

L'intégration peut se faire au niveau du gène de structure de la lévane saccharase elle-même ou dans le séquences bordant ce gène. Le gène chromosomique de la lévane saccharase est donc conservé intact. Dans tous les cas, les souches obtenues portent le gène de l'α-amylase ainsi que le gène de résistance au chloramphénicol encadrés par deux séquences identiques. Il s'agit, comme on l'a vu (figures 1 et 5) de structures amplifiables qu'on peut sélectionner par croissance en présence de concentrations croissantes de chloramphénicol.

## EXEMPLE 7

Construction d'une souche chez laquelle le gène sacB est remplacé par un gène hybride LS-amy

Les plasmides de type de ceux décrits dans l'exemple 4 peuvent s'intégrer par "crossing-over" double, ce qui provoque l'excision du gène chromosomique de la lévane saccharase. De telles souches peuvent être sélectionées par le fait qu'elles ont perdu la capacité de synthétiser cette activité enzymatique (Steinmetz et coll. 1985).

Ces constructions ne peuvent être amplifiées. On peut cependant les rendre amplifiables par transformation avec un plasmide de type de ceux décrits dans les exemples 1, 2 ou 3.

Production d'α-amylase selon le modèle de régulation de la lévane saccharase

Les souches obtenues selon les exemples 6 et 7 sont cultivées en fioles sur un milieu synthétique ayant la composition suivante :

<u>Solution A</u>

| | |
|---|---|
| Sulfate d'ammonium $(NH_4)_2SO_4$, | 3,3 g/l |
| Phosphate de potassium dipotassique $K_2HPO_4$, | 14,2 g/l |
| Phosphate de potassium monopotassique $KH_2PO_4$, | 4,1 g/l |
| Glucose, | 20,0 g/l |

<u>Solution B</u>

| | |
|---|---|
| Sulfate de magnésium $MgSO_4$, | 60 mg/l |
| Sulfate de manganèse $MnSO_4$, | 17 mg/l |
| Fer (III) citrate ammoniacal, | 22 mg/l |

Ce milieu est éventuellement additionné de saccharose inducteur (20 g/l).

De façon plus précise, la souche de B. subtilis QB 1098 utilisée dans les exemples 6 et 7 est amylase-négati ve (amyE-) avant transformation par les plasmides en cause. Elle n'exprime donc que l'α-amylase thermostable codée par ces plasmides intégrés. Par contre la souche QB 118 est amyE+.

L'activité α-amylolytique est mesurée par l'action dextrinifiante de l'enzyme. On utilise la méthode à l'iode en mesurant la densité optique à 620 nm d'une solution d'amidon à 1% (pH = 5,8) en présence d'une solution iodo-iodurée. Les unités sont exprimées en DO par rapport à un témoin sans incubation.

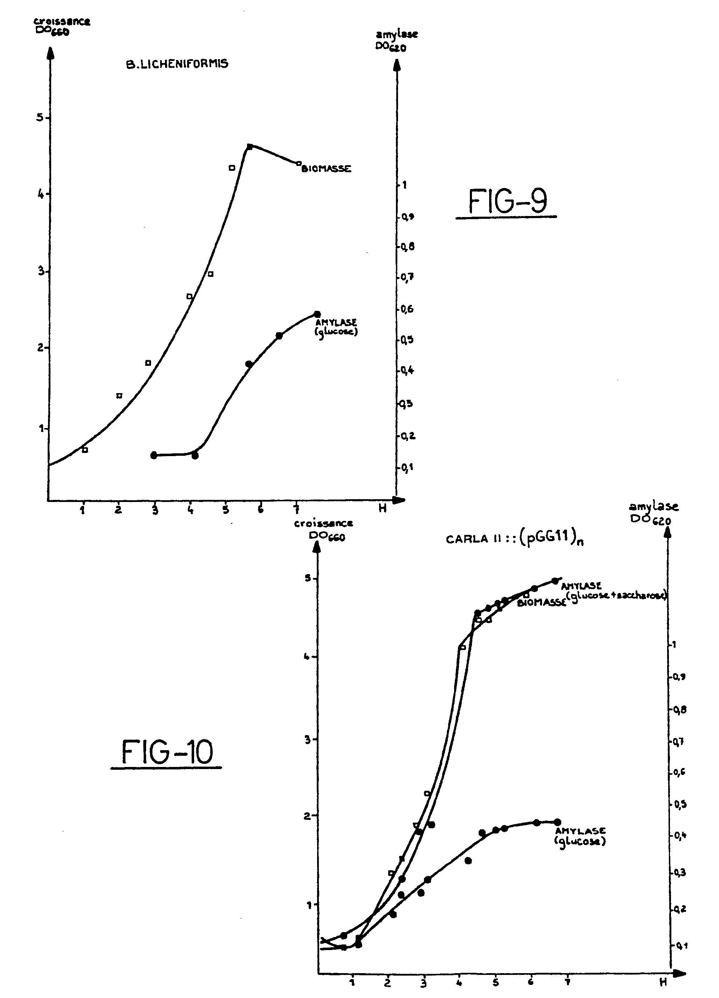

La figure 10 rapporte les résultats de deux expériences conduites en parallèle avec la même souche portant une fusion LS-α-amylase.

Cette souche, carIaII : :pGG1, est le produit de 3 étapes successives de construction définies dans l'exemple 7 :

1) une substitution du gène sacB par la fusion sacB amyθ présente sur pGG11 (exemple 4),

2) suivie d'une diploïdisation de la région substituée par transformation par pGG1, et enfin

3) l'amplification de cette région par sélection sur milieu à forte dose de chloramphénicol.

On constate qu'en présence de saccharose, l'activité α-amylase est synthétisée en phase exponentielle de croissance et suit l'augmentation de la biomasse.

On constate, par ailleurs, en l'absence de saccharose inducteur une synthèse constitutive de l'α-amylase trois fois moindre. Cette constitutivité est vraisemblablement une conséquence de l'amplification (titration du répresseur agissant au niveau de sac$^R$). On constate le même phénomène quand le gène sacB est amplifié (M. Steinmetz, observation non publiée).

La souche sauvage d'origine Bacillus licheniformis, cultivée sur le même milieu (sans saccharose) a une cinétique clairement différente, l'enzyme n'apparaissant qu'en fin de phase exponentielle et début de la phase stationnaire (figure 9).

Enfin le tableau montre les activités comparées de souches de B. subtilis que l'on a construites et qui codent pour des enzymes hybrides LS-α-amylase. Ces souches ont été cultivées sur milieu minimum glucosé, supplémenté ou non en saccharose. Les cellules ont été récoltées à DO = 1,5. L'activité amylolytique a été dosée par la méthode au DNS, après dialyse pour éliminer du milieu les sucres réducteurs, et exprimée en $DO_{545}$/mg protéine/heure.

ACTIVITES COMPAREES DE SOUCHES DE B. SUBTILIS
CODANT POUR DES ENZYMES HYBRIDES LS-α-AMYLASE

| SOUCHES | PRESENCE DE SACCHAROSE | ACTIVITE α-AMYLASE |
|---------|------------------------|--------------------|
| Carla I::(pGG11) | − | O |
| Carla I::(pGG11) | + | 172 |
| Carla II::(pGG11) | − | 188 |
| Carla II::(pGG11) | + | 895 |
| Carla III::(pGG11) | − | 170 |
| Carla III::(pGG11) | + | 191 |
| QB 118::(pGG1) | − | 543 |
| QB 118::(pGG1) | + | 1 O65 |
| QB 1098::(pGG1) | − | 450 |
| QB 1098::(pGG1) | + | 616 |

TABLEAU RESUMANT LA CONSTRUCTION DES DIFFERENTES
SOUCHES UTILISEES

QB 1098 amyE trpC$_2$ leu8

DNA QB822 Trp$^+$
+ pGG11

pGG1

select.:Cm$^s$Trp$^+$Leu$^-$

select.: Cm$^r$

CarlaI
amyE$^-$trpC$_2^+$leu8$^-$(sub. sacB/amy)

QB1098::pGG1

DNA QB112
leu$_8^+$ sacA331 sacU$_{32}^h$

select.:Trp$^+$Leu$^+$

CarlaII
amyE$^-$sacU$_{32}^h$ (sub. sacB/amy)

DNA QB174 sacS$_{32}^c$

select.: Cm$^r$

CarlaIII
amyE$^-$ sacU$_{32}^h$sacS$_{32}^c$(sub. sacB/amy)

CarlaII::pGG1

select. Cm$^r$ h. dose

CarlaII::(pGG1)$_n$

QB 118 trpC$_2$ sacU$_{32}^h$ sacR$_2^c$

pGG1

Cm$^r$

QB 118::pGG1

8

REFERENCES

1. Rosendal P., Nielsen B.H., Lange N.K. (1972) Stability of bacterial α-amylase in the starch liquefaction process. Die Stärke 31, 368-372.
2. Saïto N. (1973) A thermophilic extracellular α-amylase from Bacillus licheniformis. Archives of Biochemistry and Biophysics 155, 290-298.
3. Meers J.L. (1972) The regulation of α-amylase production in Bacillus licheniformis. Antonie van Leeuwenhoek 38, 385-390.
4. Lepensant J.A., Kunst F., Pascal M., Lepesant Kejylawa J., Steinmetz M. et Dedonder R. (1976) Specific and pleiotropic regulatory mechanisms in the sucrose system of Bacillus subtilis 168. In Microbiology, p.5859, D. Schlessinger (ed), American Society for Microbiology, Washington DC.
5. Chambert R., Petit-Glatron MF. (1984) Hyperproduction of exocellular levansucrase by B.subtilis. Examination of the phenotype of a sacU$^h$ strain. Journal of General Microbiology, 130, 3143-3152.
6. Haldewang W., Brenner C., Ollington J., Losick R., Hoch I, O'Connor M., Sonenshein A. (1980). Mapping a cloned gene under sporulation control by insertion of a drug resistance marker into the Bacillus subtilis chromosome. J. of Bacteriology, 127, 1561-1568.
7. Jannière L. Thèse de 3ème cycle, "Amplification de gènes dans le chromosome de B.subtilis". Université Pierre et Marie Curie, (1984).
8. Steinmetz M.,Le Coq D., Aymerich S., Gonzy-Treboul G.and Gay P. The DNA sequence of the gene for the secreted Bacillus subtilis enzyme levansucrase and its genetic control sites. Molec. Gen. Genetics (sous presse).
9. Stephens M.O., Ortlepp S.A., Ollington J.F. Mc.Connel D.J., Nucleotide sequence of the 5' regulatory region of the α-amylase from B.licheniformis. Comparison with the B.amyloliquefaciens gene. J. of Bacteriology, 158,369-372.
10. P. Joyet, M.Guérineau et H. Heslot (1984) Cloning of a thermostable α-amylase gene from Bacillus licheniformis and its expression in E.coli and B.subtilis. FEMS Microbiology Letters 21, 353-358.
11. Iordanescu S., Sardeanu, Della Latta P., Novick R. (1978) Incompatibility and molecular relationships between small staphylococal plasmids carrying the same resistance marker. Plasmids, 1, 468-479.

## Revendications

### Revendications pour les Etats Contractants BE-CH-DE-GB-IT-LI-LU-NL-SE

1. Vecteur d'expression et de sécrétion de l'α-amylase chez Bacillus, caractérisé en ce qu'il comporte au moins la structure :

– sacR – amy –

sacR étant le promoteur/opérateur de la lévane saccharase de B. subtilis et amy étant le gène codant pour l'α-amylase, ce gène étant sous le contrôle de sacR.

2. Vecteur selon la revendication 1, caractérisé en ce qu'il s'agit d'un plasmide auto-réplicatif dans E. coli.

3. Vecteur selon la revendication 2, caractérisé en ce que le plasmide comporte un réplicateur CollEl.

4. Vecteur selon la revendication 1, caractérisé en ce qu'il s'agit d'un vecteur d'intégration chromosomique, et en ce qu'il comporte au moins une séquence homologue du chromosome de la souche de Bacillus où il doit s'intégrer.

5. Vecteur selon la revendication 4, caractérisé en ce qu'il s'agit d'un vecteur d'intégration chromosomique ayant une structure :

– Ha – sacR – amy – Hb

Ha et Hb étant des séquences homologues du chromosome de la souche de Bacillus où il doit s'intégrer.

6. Vecteur selon l'une des revendications 1 à 5, caractérisé en ce que le locus sacR est sous forme sauvage sacR⁺.

7. Vecteur selon l'une des revendications 1 à 5, caractérisé en ce que le locus sacR est sous forme mutée sacR$^c$.

8. Vecteur selon l'une des revendications 1 à 7, caractérisé en ce qu'il comporte un gène de résistance à un antibiotique qui s'exprime dans E. coli et B. subtilis.

9. Vecteur selon l'une des revendications 1 à 8, caractérisé en ce qu'il comporte :

– sacR – sacB – amy –

sacB étant tout ou partie du gène de la lévane saccharase, le gène amy étant en phase avec le gène sacB.

10. Vecteur selon l'une des revendications 1 à 8, caractérisé en ce qu'il comporte :
$$- sacR - sacB' - c.st. - SD - amy -$$
sacB' représente une partie du gène sacB,
c.st. est le codon stop,
SD est une séquence de Shine-Dalgarno.

11. Vecteur selon l'une des revendications 1 à 10, caractérisé en ce que le gène amy est le gène amy de B. licheniformis thermostable.

12. Souche de Bacillus transformée par un vecteur selon l'une des revendications 1 à 11.

13. Souche selon la revendication 12, caractérisée en ce que le vecteur est intégré dans le chromosome de la souche de Bacillus.

14. Souche selon l'une des revendications 12 et 13, caractérisée en ce qu'un gène amy remplace tout ou partie du gène sacB dans le chromosome.

15. Souche selon la revendication 13, caractérisée en ce qu'elle a été sélectionnée sur un milieu comportant un antibiotique auquel ladite souche est devenue résistante par intégration du vecteur.

16. Souche selon la revendication 12, caractérisée en ce que le vecteur est sous forme de plasmide auto-réplicatif.

17. Procédé de préparation d'α-amylase, caractérisé en ce que l'on cultive une souche selon l'une des revendications 12 à 16 et on récupère l'α-amylase produite dans le milieu de culture en phase exponentielle de croissance.

18. Procédé selon la revendication 17, caractérisé en ce que le milieu de croissance contient un composé inducteur.

19. Procédé selon la revendication 18, caractérisé en ce que le composé inducteur est le saccharose.

## Revendications pour l'Etat Contractant AT

1. Procédé de préparation d'un vecteur d'expression et de sécrétion de l'α-amylase chez Bacillus, caractérisé en ce qu'on prépare un dit vecteur comportant au moins la structure :
$$- sacR - amy -$$
sacR étant le promoteur/opérateur de la lévane saccharase de B. subtilis et amy étant le gène codant pour l'α-amylase, ce gène étant sous le contrôle de sacR.

2. Procédé selon la revendication 1, caractérisé en ce qu'il s'agit d'un plasmide auto-réplicatif dans E. coli.

3. Procédé selon la revendication 2, caractérisé en ce que le plasmide comporte un réplicateur ColIEI.

4. Procédé selon la revendication 1, caractérisé en ce qu'il s'agit d'un vecteur d'intégration chromosomique, et en ce qu'il comporte au moins une séquence homologue du chromosome de la souche de Bacillus où il doit s'intégrer.

5. Procédé selon la revendication 4, caractérisé en ce qu'il s'agit d'un vecteur d'intégration chromosomique ayant une structure :
$$- Ha - sacR - amy - Hb$$
Ha et Hb étant des séquences homologues du chromosome de la souche de Bacillus où il doit s'intéger.

6. Procédé selon l'une des revendications 1 à 5, caractérisé en ce que le locus sacR est sous forme sauvage sacR+.

7. Procédé selon l'une des revendications 1 à 5, caractérisé en ce que le locus sacR est sous forme mutée sacR$^c$.

8. Procédé selon l'une des revendications 1 à 7, caractérisé en ce que le vecteur comporte un gène de résistance à un antibiotique qui s'exprime dans E. coli et B. subtilis.

9. Procédé selon l'une des revendications 1 à 8, caractérisé en ce que le vecteur comporte :
$$- sacR - sacB - amy -$$
sacB étant tout ou partie du gène de la lévane saccharase, le gène amy étant en phase avec le gène sacB.

10. Procédé selon l'une des revendications 1 à 8, caractérisé en ce que ledit vecteur comporte :
$$- sacR - sacR' - c.st. - SD - amy -$$
sacB' représente une partie du gène sacB,
c.st. est le codon stop,
SD est une séquence de Shine-Dalgarno.

11. Procédé selon l'une des revendications 1 à 10, caractérisé en ce que le gène amy est le gène amy de B. licheniformis thermostable.

12. Souche de Bacillu transformée par un vecteur selon l'une des revendications 1 à 11.

13. Souche selon la revendication 12, caractérisée en ce que le vecteur est intégré dans le chromosome de la souche de Bacillus.

14. Souche selon l'une des revendications 12 et 13, caractérisée en ce qu'un gène amy remplace tout ou partie du gène sacB dans le chromosome.

15. Souche selon la revendication 13, caractérisée en ce qu'elle a été selectionnée sur un milieu comportant un antibiotique auquel ladite souche est devenue résistante par intégration du vecteur.

16. Souche selon la revendication 12, caractérisée en ce que le vecteur est sous forme de plasmide auto-réplicatif.

17. Procédé de préparation d'α-amylase, caractérisé en ce que l'on cultive une souche selon l'une des revendications 12 à 16 et on récupère l'α-amylase produite dans le milieu culture en phase exponentielle de croissance.

18. Procédé selon la revendication 17, caractérisé en ce que le milieu de croissance contient un composé inducteur.

19. Procédé selon la revendication 18, caractérisé en ce que le composé inducteur est le saccharose.

## Ansprüche

### Patentansprüche für die Vertragstraaten BE, CH, DE, GB, IT, LI, LU, NL, SE

1. Expressions- und Sekretionsvektor von α-amylase in Bacillus, dadurch gekennzeichnet, daß es mindestens die Struktur :

– sacR – amy –

umfaßt, wobei sacR der Promotor/Operator von Lévane Saccharase von B. subtilis ist und wobei amy der Gencode für α-amylase ist, wobei dieses Gen unter der Kontrolle von sacR ist.

2. Vektor nach Anspruch 1, dadurch gekennzeichnet, daß es sich um ein auto-replizierendes Plasmid in E. coli handelt.

3. Vektor nach Anspruch 2, dadurch gekennzeichnet, daß das Plasmid einen Replikator CollEl umfaßt.

4. Vektor nach Anspruch 1, dadurch gekennzeichnet, daß es sich um einen Chromosomen-Integrationsvektor handelt und daß er mindestens eine homologe Chromosomensequenz aus dem Stamm Bacillus umfaßt, wo er sich integrieren soll.

5. Vektor nach Anspruch 4, dadurch gekennzeichnet, daß es sich um einen Chromosomen-Integrationsvektor handelt mit einer Struktur :

– Ha – sacR – amy – Hb

wobei Ha und Hb homologe Chromosomensequenzen aus dem Stamm Bacillus sind, wo er sich integrieren soll.

6. Vektor nach einem der Ansprüche 1 bis 5, dadurch gekennzeichnet, daß der Locus sacR in Form von rohem sacR⁺ vorliegt.

7. Vektor nach einem der Ansprüche 1 bis 5, dadurch gekennzeichnet, daß der Locus sacR in Form von mutiertem sacRᶜ vorliegt.

8. Vektor nach einem der Ansprüche 1 bis 7, dadurch gekennzeichnet, daß er umfaßt ein gegenüber einem Antibiotikum resistentes Gen, das sich in E. coli und B. subtilis exprimiert.

9. Vektor nach einem der Ansprüche 1 bis 8, dadurch gekennzeichnet, daß er umfaßt :

– sacR – sacB – amy –

wobei sacB ganz oder ein Teil des Gens der Lévane Saccharase ist, wobei das Gen amy in einer Stufe mit dem Gen sacB ist.

10. Vektor nach einem der Ansprüche 1 bis 8, dadurch gekennzeichnet, daß er umfaßt :

– sacR – sacB' –c.st.– SD – amy –

sacB' stellt einen Teil des Gens sacB dar, c.st. ist der Codonstop, SD ist eine Sequenz des Shine-Dalgarno.

11. Vektor nach einem der Ansprüche 1 bis 10, dadurch gekennzeichnet, daß das Gen amy das Gen amy von wäremebeständigen B. licheninformis ist.

12. Stamm Bacillus transformiert durch einen Vektor nach einem der Ansprüche 1 bis 11.

13. Stamm nach Anspruch 12, dadurch gekennzeichnet, daß der Vektor integriert ist in dem Chromosom des Stamms Bacillus.

14. Stamm nach einem der Ansprüche 12 und 13, dadurch gekennzeichnet, daß ein Gen amy ganz oder teilweise das Gen sacB im Chromosom ersetzt.

15. Stamm nach Anspruch 13, dadurch gekennzeichnet, daß er ausgewählt wird aus einem Milieu umfassend ein Antibiotikum, worin der darin ausgesetzte Stamm resistent wird durch Integration des Vektors.

16. Stamm nach Anspruch 12, dadurch gekennzeichnet, daß der Vektor in Form von einem auto-replizierenden Plasmid vorliegt.

17. Verfahren zur Herstellung von α-amylase, dadurch gekennzeichnet, daß ein Stamm nach einem der Ansprüche 12 bis 16 kultiviert wird und die hergestellte α-amylase in dem Kulturmilieu in dem Stadium exponentiellen Wachstums gewonnen wird.

18. Verfahren nach Anspruch 17, dadurch gekennzeichnet, daß das Wachstumsmilieu eine Induktorzusammensetzung enthält.

19. Verfahren nach Anspruch 18, dadurch gekennzeichnet, daß die Induktorzusammensetzung Saccharose ist.

## Patentansprüche für den Vertragstaat AT

1. Verfahren zur Herstellung eines Expressions- und Sekretionsvektor von α-amylase in <u>Bacillus</u>, dadurch gekennzeichnet, daß ein solcher Vektor hergestellt und umfasen mindestens die Struktur :
$$- \underline{sacR} - \underline{amy} -$$
wobei sacR der Promotor/Operator von Lévane Saccharase von <u>B. subtilis</u> ist und wobei <u>amy</u> der Gencode für α-amylase ist, wobei dieses Gen unter der Kontrolle von <u>sacR</u> ist.

2. Verfahren nach Anspruch 1, dadurch gekennzeichnet, daß es sich um ein auto-replizierendes Plasmid in <u>E. coli</u> handelt.

3. Verfahren nach Anspruch 2, dadurch gekennzeichnet, daß das Plasmid einen Replikator CollEl umfaßt.

4. Verfahren nach Anspruch 1, dadurch gekennzeichnet, daß es sich um einen Chromosomen-Integrationsvektor handelt und daß er mindestens eine homologe Chromosomensequenz aus dem Stamm Bacillus umfaßt, wo er sich integrieren soll.

5. Verfahren nach Anspruch 4, dadurch gekennzeichnet, daß es sich um einen Chromosomen-Integrationsvektor handelt mit einer Struktur :
$$- Ha - \underline{sacR} - \underline{amy} - Hb$$
wobei Ha und Hb homologe Chromosomensequenzen aus dem Stamm Bacillus sind, wo er sich integrieren soll.

6. Verfahren nach einem der Ansprüche 1 bis 5, dadurch gekennzeichnet, daß der Locus <u>sacR</u> in Form von rohem <u>sacR$^+$</u> vorliegt.

7. Verfahren nach einem der Ansprüche 1 bis 5, dadurch gekennzeichnet, daß der Locus <u>sacR</u> in Form von mutiertem <u>sacR$^c$</u> vorliegt.

8. Verfahren nach einem der Ansprüche 1 bis 7, dadurch gekennzeichnet, daß er umfaßt ein gegenüber einem Antibiotikum resistentes Gen, das sich in <u>E. coli</u> und <u>B. subtilis</u> exprimiert.

9. Verfahren nach einem der Ansprüche 1 bis 8, dadurch gekennzeichnet, daß er umfaßt :
$$- \underline{sacR} - \underline{sacB} - \underline{amy} -$$
wobei <u>sacB</u> ganz oder ein Teil des Gens der Lévane Saccharase ist, wobei das Gen <u>amy</u> in einer Stufe mit dem Gen <u>sacB</u> ist.

10. Verfahren nach einem der Ansprüche 1 bis 8, dadurch gekennzeichnet, daß er umfaßt :
$$- \underline{sacR} - \underline{sacB}' - c.st. - SD - \underline{amy} -$$
sacB' stellt einen Teil des Gens <u>sacB</u> dar, c.st. ist der Codonstop, SD ist eine Sequenz des Shine-Dalgarno.

11. Verfahren nach einem der Ansprüche 1 bis 10, dadurch gekennzeichnet, daß das Gen <u>amy</u> das Gen <u>amy</u> von wäremebeständigen <u>B. licheninformis</u> ist.

12. Stamm Bacillus transformiert durch einen Vektor nach einem der Ansprüche 1 bis 11.

13. Stamm nach Anspruch 12, dadurch gekennzeichnet, daß der Vektor integriert ist in dem Chromosom des Stamms <u>Bacillus</u>.

14. Stamm nach einem der Ansprüche 12 und 13, dadurch gekennzeichnet, daß ein Gen <u>amy</u> ganz oder teilweise das Gen <u>sacB</u> im Chromosom ersetzt.

15. Stamm nach Anspruch 13, dadurch gekennzeichnet, daß er ausgewählt wird aus einem Milieu umfassend ein Antibiotikum, worin der darin ausgesetzte Stamm resistent wird durch Integration des Vektors.

16. Stamm nach Anspruch 12, dadurch gekennzeichnet, daß der Vektor in Form von einem auto-replizierenden Plasmid vorliegt.

17. Verfahren zur Herstellung von α-amylase, dadurch gekennzeichnet, daß ein Stamm nach einem der Ansprüche 12 bis 16 kultiviert wird und die hergestellte α-amylase in dem Kulturmilieu in dem Stadium exponentiellen Wachstums gewonnen wird.

18. Verfahren nach Anspruch 17, dadurch gekennzeichnet, daß das Wachstumsmilieu eine Induktorzusammensetzung enthält.

19. Verfahren nach Anspruch 18, dadurch gekennzeichnet, daß die Induktorzusammensetzung Saccharose ist.

## Claims

### Claims for the Contracting States BE, CH, DE, GB, IT, LI, LU, NL, SE

1. Vector for the expression and secretion of α-amylase in Bacillus, characterized in that it contains at least the structure :

$$- sacR - amy -$$

sacR being the promoter/operator of B. subtilis levansucrase and amy being the gene coding for α-amylase, this gene being under the control of sacR.

2. Vector according to Claim 1, characterized in that it is a plasmid which is self-replicating in E. coli.

3. Vector according to Claim 2, characterized in that the plasmid contains a ColEI replicator.

4. Vector according to Claim 1, characterized in that it is a chromosomal integration vector, and in that it contains at least one sequence homologous to the chromosome of the Bacillus strain in which it is to be integrated.

5. Vector according to Claim 4, characterized in that it is a chromosomal integration vector having a structure :

$$- Ha - sacR - amy - Hb$$

Ha and Hb being sequences homologous to the chromosome of the Bacillus strain in which it is to be integrated.

6. Vector according to one of Claims 1 to 5, characterized in that the sacR locus is in sacR$^+$ wild-type form.

7. Vector according to one of Claims 1 to 5, characterized in that the sacR locus is in sacR$^c$ mutated form.

8. Vector according to one of Claims 1 to 7, characterized in that it contains an antibiotic resistance gene which is expressed in E. coli and B. subtilis.

9. Vector according to one of Claims 1 to 8, characterized in that it contains :

$$- sacR - sacB - amy -$$

sacB being all or part of the levansucrase gene, the amy gene being in phase with the sacB gene.

10. Vector according to one of Claims 1 to 8, characterized in that it contains :

$$- sacR - sacB' - c.st. - SD - amy -$$

sacB' represents a portion of the sacB gene, c.st. is the stop codon, SD is a Shine-Dalgarno sequence.

11. Vector according to one of Claims 1 to 10, characterized in that the amy gene is the thermostable B. licheniformis amy gene.

12. Bacillus strain transformed by a vector according to one of Claims 1 to 11.

13. Strain according to Claim 12, characterized in that the vector is integrated in the chromosome of the Bacillus strain.

14. Strain according to one of Claims 12 and 13, characterized in that an amy gene replaces all or part of the sacB gene in the chromosome.

15. Strain according to Claim 13, characterized in that it has been selected on a medium containing an antibiotic to which the said strain has become resistant by integration of the vector.

16. Strain according to Claim 12, characterized in that the vector is in the form of a self-replicating plasmid.

17. Process for preparing α-amylase, characterized in that a strain according to one of Claims 12 to 16 is cultured and the α-amylase produced in the culture medium in the exponential growth phase is recovered.

18. Process according to Claim 17, characterized in that the growth medium contains an inducing compound.

19. Process according to Claim 18, characterized in that the inducing compound is sucrose.

### Claims for the Contracting State AT

1. Process for preparing a vector for the expression and secretion of α-amylase in Bacillus, charac-

terized in that a said vector is prepared containing at least the structure :

$$- sacR - amy -$$

sacR being the promoter/operator of B. subtilis levansucrase and amy being the gene coding for α-amylase, this gene being under the control of sacR.

2. Process according to Claim 1, characterized in that the vector is a plasmid which is self-replicating in E. coli.

3. Process according to Claim 2, characterized in that the plasmid contains a ColEI replicator.

4. Process according to Claim 1, characterized in that the vector is a chromosomal integration vector, and in that it contains at least one sequence homologous to the chromosome of the Bacillus strain in which it is to be integrated.

5. Process according to Claim 4, characterized in that the vector is a chromosomal integration vector having a structure :

$$- Ha - sacR - amy - Hb$$

Ha and Hb being sequences homologous to the chromosome of the Bacillus strain in which it is to be integrated.

6. Process according to Claims 1 to 5, characterized in that the sacR locus is in sacR$^+$ wild-type form.

7. Process according to one of Claims 1 to 5, characterized in that the sacR locus is in sacR$^c$ mutated form.

8. Process according to one of Claims 1 to 7, characterized in that the vector contains an antibiotic resistance gene which is expressed in E. coli and B. subtilis.

9. Process according to one of Claims 1 to 8, characterized in that the vector contains :

$$- sacR - sacB - amy -$$

sacB being all or part of the levansucrase gene, the amy gene being in phase with the sacB gene.

10. Process according to one of Claims 1 to 8, characterized in that the said vector contains :

$$- sacR - sacR' - c.st. - SD - amy -$$

sacB' represents a portion of the sacB gene, c.st. is the stop codon, SD is a Shine-Dalgarno sequence.

11. Process according to one of Claims 1 to 10, characterized in that the amy gene is the thermostable B. licheniformis amy gene.

12. Bacillus strain transformed by a vector according to one of Claims 1 to 11.

13. Strain according to Claim 12, characterized in that the vector is integrated in the chromosome of the Bacillus strain.

14. Strain according to one of Claims 12 and 13, characterized in that an amy gene replaces all or part of the sacB gene in the chromosome.

15. Strain according to Claim 13, characterized in that it has been selected on a medium containing an antibiotic to which the said strain has become resistant by integration of the vector.

16. Strain according to Claim 12, characterized in that the vector is in the form of a self-replicating plasmid.

17. Process for preparing α-amylase, characterized in that a strain according to one of Claims 12 to 16 is cultured and the α-amylase produced in the culture medium in the exponential growth phase is recovered.

18. Process according to Claim 17, characterized in that the growth medium contains an inducing compound.

19. Process according to Claim 18, characterized in that the inducing compound is sucrose.

14

A

INTEGRATION

B

AMPLIFICATION

→ région d'homologie

ADN chromosomique

α-amylase

ADN plasmidique

FIG-1

1:Restriction Pst I

2:Action de la DNAse S1

3:Restriction Hind III

1:Restriction Cla1

2:Action du fragment de Klenow

3:Restriction Hind III

4:Ligation

## FIG-2

FIG-3

FIG-4

FIG-5

FIG-6

## FIG-7

amylase hybride

amylase de Bacillus licheniformis

amylase de Bacillus subtilis

## FIG-8

FIG-9

FIG-10